# FASCICULE DE BREVET EUROPEEN

(11) **EP 1 023 264 B1**
(45) Date de publication et mention de la délivrance du brevet: **19.03.2003**
(21) Numéro de dépôt: 96942986.9
(22) Date de dépôt: 11.12.1996
(51) Int. Cl.: C07D 203/10, C07B 59/00, G01N 33/60, G01N 33/574, G01N 33/534

(54) **NOUVEAU MARQUEUR, SON PROCEDE D'OBTENTION ET SON UTILISATION**
MARKER, HERSTELLUNGSVERFAHREN UND DESSEN VERWENDUNG
NEW MARKER, PRODUCTION PROCESS AND USE THEREOF

(30) Priorité: 12.12.1995 BE 9501028
(43) Date de publication de la demande: 02.08.2000
(73) Titulaire: Quivy, Jacques, 1348 Louvain-la-Neuve (BE)
(72) Inventeur: Quivy, Jacques, 1348 Louvain-la-Neuve (BE)
(86) Numéro de dépôt international: BE9600130
(87) Numéro de publication internationale: WO97021674

(56) Documents cités:
- EP-A- 0 260 066
- JOURNAL OF LABELLED COMPOUNDS AND RADIOPHARMACEUTICALS, vol. XXXIV, no. 3, 1993, pages 297-302, XP000566289 R. MCCAGUE ET AL: "Synthesis of 4-stannylated tamoxifen analogues; useful precursors to radiolabelled idoxifene and aziridinyl 4-iodotamoxifen" cité dans la demande
- STEROIDS, vol. 48, no. 5-6, 1986, pages 287-313, XP000565745 F. G. SALITURO ET AL: "125I-Iododesethyl tamoxifen aziridine: synthesis and covalent labeling of the estrogen receptor with an iodine-labeled affinity label" cité dans la demande

## Description

La présente invention concerne l'isomère trans du 4-iodotamoxifen-aziridine ou E-1-(4-[2-N-(aziridyl)-éthoxy]phényl)-1-(4-iodophényl)-2-phényl-1-butène, sa préparation et son utilisation.

Celui-ci répond à la formule :

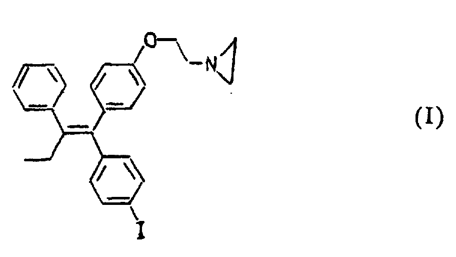

Le marquage des cytosols propres au cancer du sein humain à l'aide de tamoxifen aziridine (TAZ) tritié a mis en évidence un polymorphisme moléculaire très important des récepteurs d'oestrogène. Il est possible d'identifier ainsi le polymorphisme des récepteurs d'oestrogène de chaque tumeur (S. TRIVEDI et coll. Breast Cancer Research and Treatment 40, 231-241, 1996).

Ce type d'analyse se trouve toutefois limité par la très faible activité spécifique du TAZ tritié (∼25 Ci/mmole).

On sait par ailleurs, voir publication de R. McCAGUE et G. A. POTTER parue dans le Journal of Labelled Compounds and Radiopharmaceuticals - Vol. XXXIV, N° 3, pages 297-302, qu'on peut synthétiser des analogues du tamoxifen stannylés en position 4 en tant que précurseurs de composés iodés radiomarqués. La publication décrit en particulier un composé (E)-1-[4-[2-(aziridin-1-yl) éthoxy]phényl]-1-[4-(tributylstannyl)phényl]-2-phényl-1-butène ou 4-tributylétaintamoxifen-aziridine, de formule :

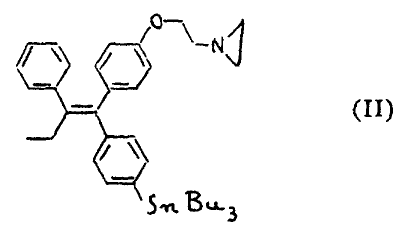

La publication décrit la préparation de ce composé et suggère son utilisation comme précurseur du 4-iodo-tamoxifen, en supposant que le produit iodé radiomarqué pourrait se lier aux récepteurs des oestrogènes.

Si l'on considère les modalités de fixation du récepteur oestrogène, on peut cependant douter de l'utilité du composé iodé radioactif pour le marquage de ces récepteurs, en raison de l'encombrement stérique de l'iode et par le fait que celui-ci ne peut pas réaliser les ponts hydrogènes intervenant dans la fixation de l'hormone au récepteur.

La publication de SALITURO et coll., parue dans Stéroïds 48/5-6 Novembre-décembre 1986, pp 287-313, décrit un dérivé de tamoxifen aziridine iodé en position 2, obtenu sous forme de mélange d'isomères cis/trans dont il faut séparer l'isomère trans qui seul reconnaît le récepteur oestrogène.

La présente invention part de la constatation qu'il est possible d'obtenir le 4-iodotamoxifen sous forme d'isomère trans pur et que l'activité spécifique de cet isomère est plus élevée que celle du dérivé 2-iodé décrit par SALITURO et coll. et nettement plus élevée que celle du tamoxifen aziridine tritié mentionné précédemment.

L'invention a donc pour objet le produit marqué du trans-4-iodo-tamoxifenaziridine tel que défini aux revendications 1 et 4, sa préparation telle que définie aux revendications 2 et 3 et son utilisation telle que définie aux revendications 5 à 8.

Plus particulièrement, le procédé pour introduire l'iode dans la molécule par déstannylation du précurseur au tributylétain se distingue des procédés connus par le fait qu'il met en oeuvre l'ion I⁽⁻⁾ à pH acide non tamponné en réduisant I⁽⁺⁾ par Na₂S. Ainsi, tout l'iode est présent sous forme de I⁽⁻⁾ avant d'être oxydé en I⁽⁺⁾ par la chloramine T en vue de sa fixation à la molécule de tamoxifen-aziridine. Il n'y a, avant cette étape de fixation, aucune forme *I⁽⁺⁾ qui pourrait se recombiner avec *I⁽⁻⁾ pour former *I₂ et s'échapper dans l'atmosphère et contaminer les lieux.

### Exemple de réalisation :

Obtention du trans-4-iodotamoxifen-aziridine (E-1-[4-[2-N-(aziridyl)-éthoxy]phényl]-1-(4-iodophényl)-2-phényl-1-butène) marqué par l'iode 125.

Un mélange de 10 microlitres d'une solution de Na¹²⁵I (1mCi, IMS30 de AMERSHAM), 2 microlitres d'une solution de Na₂S (0,1 mg/ml) dans l'éthanol et 4 microlitres d'une solution aqueuse d'HCL 0,1N est placé dans une ampoule à visser en matière plastique, ensuite est agité vigoureusement quelques secondes et laissé à température ambiante pendant 5 minutes.

On ajoute ensuite au mélange, 20 microlitres d'éthanol absolu, 10 microlitres du précurseur 4-tributylétaintamoxifen aziridine (décrit dans l'article de McCAGUE et POTIER publié dans le Journal of Labelled Compounds and Radiopharmaceuticals vol. 34 N° 3, pages 297-302) en solution dans l'éthanol (1,2 mg/ml), et 10 microlitres d'une solution de Chloramine T (1mg/mℓ) dans le méthanol. Ensuite, on agite vigoureusement l'ensemble quelques secondes avant de laisser réagir pendant 8 minutes.

Après ce délai, la réaction est stoppée par l'ajout de 8 microtitres de Na₂S en solution dans l'eau (10mg/ml).

Le mélange est ensuite injecté dans un système HPLC (chromatographie liquide à haute pression) avec une colonne phase inverse µBondapack® C18 4,6x300mm ; une phase mobile EtOH-H₂O, 85/15, v/v ; et un débit de 0,8ml/min. Le pic d'iodotamoxifen aziridine (ITAZ) marqué à l'iode 125 est élué entre 11-14min, et collecté (activité spécifique calculée par UV absorbance en ligne dans HPLC : 1800 à 2000 Ci/mmol ; rendement radiochimique : 70-90%).

Ce pic marqué d'iodotamoxifen aziridine a le même temps de rétention qu'un aliquote de standard iodé non radioactif préparé par la même méthode où l'iode est non-radioactif (¹²⁷I) et caractérisé par RMN, SM, IR, etc. Le pic correspondant à l'isomère '"cis" marqué est très faible et non quantifiable.

### Exemples d'application en biologie

### EXEMPLE 1.

Méthode de marquage du récepteur aux oestrogènes (RO) dans des extraits cytosoliques.

Les extraits cytosoliques sont dilués dans leur tampon d'homogénéisation de manière à obtenir des concentrations en protéines de l'ordre de 1-1,5mg/ml. Cinq cents microlitres du cytosol auquel on aura préalablement ajouté 100µl de 2,5M KCl (concentration finale 0,5M) et 35 µl de DMF (concentration finale de 7%) sont alors incubés à 0°C pendant 90 minutes en présence de [¹²⁵I] -TAZ de façon à obtenir une concentration finale de 1nM (+/- 2,3 µl selon l'activité spécifique qui peut varier d'un échantillon à l'autre). Au terme de l'incubation, le ligand iodé non lié est éliminé au moyen du DDC concentré (dextran 0,5g, charbon actif 5g dans un tampon phosphate 10mM pH 7,4 ; 50µl). La suspension est ensuite centrifugée à 3500g à 4°C pendant 10 minutes. Le surnageant représente la préparation marquée du RO.

Pour permettre la comparaison avec un produit existant, les extraits sont marqués au [³H]-TAZ, le marquage du RO s'effectue selon le même protocole expérimental (concentration finale de [³H]-TAZ : 20 nM).

A 500 µl de la préparation ainsi marquée, on ajoute 1 µl de l'AC monoclonal anti-RO (H-222 ou H-226 - 1,0 mg/ml) qu'on laisse incuber 2 heures à 0-4°C. Après addition de 50 µl d'IgG-agarose anti-rat (l'équilibration de l'AC secondaire se fait selon les instructions du fabricant, Sigma), les échantillons sont incubés sur un roller-mixer pendant la nuit. La fraction immunoprécipitée (culot) obtenue par centrifugation (3500g durant 15 minutes à 0°C) est lavée à 3 reprises avec du tampon 20mM Tris-HCl contenant du KCl à 500 mM ; pH 7,4. Le culot ainsi traité est ensuite suspendu dans 100 µl d'un tampon de lyse.

La fraction non immunoprécipitée (surnageant)est mélangée à ce tampon de lyse à volume égal. Les culots et les surnageants sont ensuite dénaturés dans un bain d'eau bouillante pendant 5 minutes afin d'en extraire le RO marqué.

Les échantillons cytosoliques marqués par ¹²⁵I Iodotamoxifen aziridine sont analysés par la technique d'électrophorèse sur gel de polyacrylamide (SDS-PAGE).

Les extraits marqués sont déposés dans les puits à raison de 35 µl de même que 10 µl d'un témoin dont la masse moléculaire est connue. Les puits sont ensuite délicatement remplis de tampon d'électrophorèse. Ces supports sont placés dans les cuves à électrophorèse. Du tampon d'électrophorèse est ajouté ainsi que du bleu de bromophénol. Les cuves sont alimentées en courant (ddp=200V et I=25mA par gel).

Après l'électrophorèse, les gels sont déposés dans une solution colorante de bleu de Coomasie (bleu de Coomasie 0,08%, méthanol 10%, acide acétique 5%). On procède ensuite à leur séchage dans une feuille de cellophane pendant 2 heures à 80°C Les bandes radioactives correspondantes au complexe RO ~[¹²⁵I]-TAZ sont visualisées par exposition de 1 à 2 jours à 80°C à un film photographique (X ray OMAT®-Kodak). Le poids moléculaire correspondant aux formes du RO est évalué à l'aide des standard (Pharmacia), soumis à l'électrophorèse en parallèle avec le RO.

Par comparaison, lorsque les extraits sont marqués par le ligand tritié ( les gels après coloration, sont déposés dans du En³Hance® durant 30 minutes et séchés), une période d'exposition de 21 jours est requise pour la révélation des bandes du complexe RO ~[³H]-TAZ.

### Résultats expérimentaux et avantages du nouveau Ligand iodé :

### Efficience du [¹²⁵I]-TAZ dans le marquage du RO préalablement à son immunoabsorption par l'AC. monoclonal anti-RO H-222.

### Sensibilité de la sonde iodée

Dans un premier temps, une série d'expériences fut réalisée sur un RO humain recombinant (hER).

Diverses concentrations de [¹²⁵I]-TAZ furent utilisées lors du marquage. En présence de 0,1 nM du ligand, on visualise une bande de 67 Kda particulièrement intense après seulement 2 jours d'exposition du gel d'électrophorèse au film photographique. Cette bande est partiellement supprimée lorsque l'échantillon est mis en présence d'un excès d'oestradiol (1µM) préalablement au marquage par le [¹²⁵I]-TAZ. Ceci est un argument en faveur de sa spécificité (compétition entre l'oestradiol et le TAZ pour le RO).

De manière inattendue, de très faibles concentrations du ligand (de l'ordre de 0,01nM) permettent toujours la visualisation du récepteur dans les mêmes conditions d'exposition. Notons également que lorsque l'on double les temps d'exposition du gel au film photographique (4 jours), on peut visualiser le récepteur en présence d'une quantité encore moindre du marqueur (1pM). A titre comparatif, signalons qu'en présence de TAZ tritié, la mise en évidence de peptides marqués de manière analogue requiert des concentrations beaucoup plus importantes (de l'ordre de 20nM) et surtout un temps d'exposition 10 fois plus long (environ trois semaines).

### EXEMPLE 2

Méthode de marquage de la protéine membranaire P-gp associée au MDR (syndrome de "multi-drug resistance" de certaines cellules tumorales).

Les cellules utilisées en culture, sont des cellules de poumons de hamster chinois DC-3F et de leur variant DC-3F/VCRd-5L (résistant à la vincristine). Ces dernières sont maintenues en présence de vincristine jusque 1 semaine avant l'expérience de marquage.

### Marquage proprement-dit :

Des cellules, en phase de croissance exponentielle, sont collectées à l'aide d'un "rubber blade". Les cellules viables au Bleu Tripan (>90%) sont mises en suspension (500.000 cellules/essai) dans du milieu tampon phosphate-Dulbecco sans Ca2 +-Mg2+, en présence d'iodotamoxifen aziridine (I) marquée à l'iode 125 (1 à 100nM), avec ou sans le 4-hydroxytamoxifen pendant 90 minutes.

Les cellules sont solubilisées au détergent et immunoprécipitées (5 millions de cellules marquées par essai) selon une méthode décrite par Safa et coll. J. Bio. Chem. 261, 6137-6140 (1986). Les échantillons d'extraits cellulaires marqués par [¹²⁵I]-ITAZ (I) sont ensuite analysés par SDS-PAGE et révélés par autoradiographie (voir exemple 1).

### RESULTATS

Le marquage des cellules DC-3F et leur variant DC-3F/VCRd type MDR par [¹²⁵I]-ITAZ suivi par SDS-PAGE et autoradiographie révèle un certain nombre de protéines marquées. Toutefois, une protéine de 150-180 kDa est marquée préférentiellement dans le variant cellulaire DC-3F/VCRd type MDR.

L'immunoprécipitation avec l'anti-corps monoclonal C219 anti-P-gp suivie de SDS-PAGE, montre que la bande marquée et révélée par autoradiographie qui correspond à une protéine de 150-180 kDa, est bien la protéine P-gp.

Quand le marquage est réalisé conjointement avec le 4-hydroxytamoxifen (10 µM) cette bande marquée est fortement atténuée; ce qui démontre la spécificité du marquage.

## Revendications

1. Trans-4-iodotamoxifen-aziridine ou trans-E-1-[4-[2-N-(aziridyl)-ethoxy]phenyl]-1-(4-iodophenyl)-2-phenyl-1-butène de formule I : 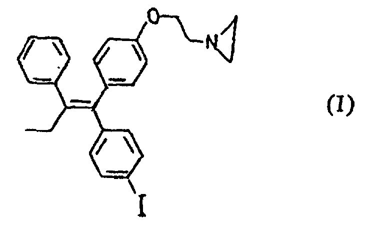 marqué à l'iode 125 et possédant une activité spécifique de 1800-2000 Ci/mmole.

2. Procédé d'obtention du composé de formule I marqué à l'iode 125 selon la revendication 1,
**caractérisé en ce qu'**on traite le composé de formule II 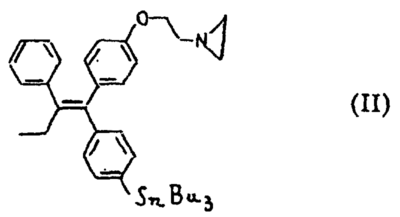 par de l'iode 125, en milieu réducteur, non tamponné, obtenu par un mélange de Na¹²⁵I et de Na₂S, à un pH acide, avant de mettre le mélange en présence de chloramine T.

3. Procédé selon la revendication 2,
**caractérisé en ce que** le milieu de réaction non tamponné se trouve à un pH d'environ 2, ou de préférence d'environ 2,5.

4. Le produit selon la revendication 1 lorsqu'il est marqué par un autre isotope radioactif de l'iode, soit l'iode 123 ou l'iode 124 ou l'iode 131.

5. Utilisation du produit marqué selon la revendication 1 en biochimie, pour le marquage de protéines qui lient les oestrogènes.

6. Utilisation du produit marqué selon la revendication 1 pour le marquage de protéines utilisables en diagnostic in vitro.

7. Utilisation du produit marqué selon la revendication 1 pour déterminer le polymorphisme du récepteur oestrogène dans le cancer du sein.

8. Utilisation du produit marqué selon la revendication 1 pour le marquage des protéines liant le tamoxifen et ses dérivés, c'est-à-dire le récepteur aux oestrogènes et ses différentes formes, ou la protéine membranaire P-gp impliquée dans le MDR (phénomène de résistance aux agents anti-cancéreux).

## Patentansprüche

1. Trans - 4 - jodotamoxifen - aziridine ou trans - E - 1 - [4 - [2- N -( aziridyl ) - ethoxy ] phenyl ] - 1 - ( 4- jodophenyl ) - 2 - phenyl - 1 - butene von Formel I : 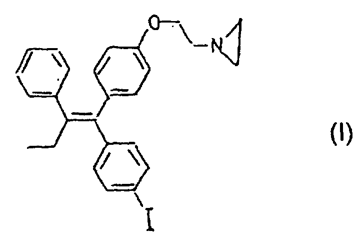 an Jod 125 markiert und in Besitz einer spezifischen Wirkung von 1800 - 2000 Ci/mmole

2. Ergänzungverfahren der Zusammensetzung vom Formel I mit Jod 125 nach Anspruch 1 charakterisiert beim verfahren der Zusammensetzung vom Formel II : 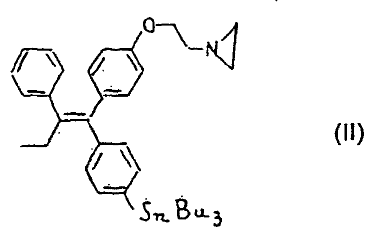 durch Jod 125 in eine reduzierende ungepuffert Umgebung durch eine Mishung von Na ¹²⁵J und Na₂S bekommen. Die vergenannte Mischung soll ein pH sauer zeigen eher ihre Vorstellung mit Chloramine T.

3. Verfahren nach Anspruch 2 durch der tat dass die ungepuffert Reaktionsumgebung sich befindet bei ein pH von ungefähr 2 oder besser ungefähr 2,5.

4. Das Produkt nach Anspruch 1 wenn es durch einen anderen radioaktives Isotop von Jod, des Jod 123 oder Jod 124 oder Jod 131 markiert wird.

5. Die Verwendung nach Anspruch 1 des markiert Produkt, in Biochemie für die Markierung von Proteinen in Bindung mit Östrogenen.

6. Die Verwendung nach Anspruch 1 des markierte Produkt für die Markierung von Proteinen im Falle von Diagnose "in vitro".

7. Die Verwendung nach Anspruch 1 des markiert Produkt für die Bestimmung des Polymorphism des Östrogen-Rezeptor im Falle eines Brustkrebs.

8. Die Verwendung nach Anspruch 1 des markiert Produkt für die Markierung von Proteinen bindend Tamoxifen und seine Derivaten, es ist zu sagen der Östrogen-Rezeptor sowie die verschiedene Formen, oder die Zellmembrane Protein P-gp in der MDR (Phänomen von Wiederstand an den Anti-Krebs Agenten) einbegriffen.

## Claims

1. Trans - 4 - iodotamoxifen - aziridine ou trans - E - 1 - [ 4 - [ 2 - N -( aziridyl ) - ethoxy ] phenyl] - 1 - ( 4- iodophenyl ) - 2 - phenyl - 1 - butene of Formula I : 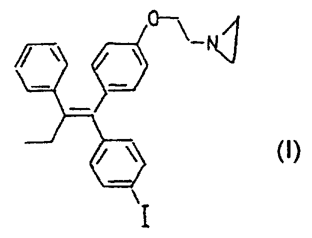 labelled with iodine 125 and possessing specific activity of 1800- 2000 Ci/mmol.

2. Production process of compound of formula I labelled with iodine 125 according to Claim 1, **characterized in that** the compound of formula II : 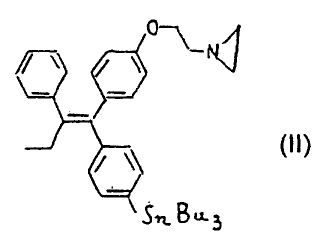 Is treated with iodine 125 in reducing medium and unbuffered, obtained from a mixture of Na ¹²⁵I and Na₂S in acidic pH, before to put this mixture in the presence of chloramine T.

3. Process according to Claim1 **characterized in that** the reaction mixture unbuffered is at pH near 2 or better near 2.5.

4. Product according to Claim 1 when is labelled with an other radioactive isotope of iodine choosen from iodine 123, iodine 124 or iodine 131.

5. Use of labelled product according to claim 1 in biochemistry, for the labelling of proteins binding estrogens.

6. Use of labelled product according to claim 1 for the labelling of proteins useful for "in vitro" diagnostics.

7. Use of labelled product according to claim 1 to determine the polymorphism of estrogen receptor in breast cancer disease.

8. Use of labelled product according to claim 1 for the labelling of proteins binding tamoxifen and its derivatives so, estrogens receptor and its different forms, or the membrane protein P-gp implicated in MDR ( anticancer multi-drug resistance).
